# EUROPEAN PATENT APPLICATION

(11) **EP 1 704 823 A1**
(43) Date of publication of application: **27.09.2006**
(21) Application number: 06005556.3
(22) Date of filing: 17.03.2006
(51) Int. Cl.: A61B 17/88, A61B 17/00, A61F 2/44, A61F 2/02

(54) **Leakage prevention bag for injecting and filling bone cement**

(30) Priority: 24.03.2005 JP 2005086225
(71) Applicant: GC Corporation, Tokyo 174-8585 (JP); Nippon Sigmax Co., Ltd., Shinjuku-ku Tokyo 163-6033 (JP)
(72) Inventor: Kaneko, Tadashi, GC Corporation, Itabashi-ku Tokyo 174-8585 (JP); Yamamoto, Katsushi, GC Corporation, Itabashi-ku Tokyo 174-8585 (JP); Takahashi, Masaru, Nippon Sigmax Co.,Ltd, Shinjuku-ku Tokyo 163-6033 (JP)
(74) Representative: Müller-Boré & Partner Patentanwälte

(57) **Abstract**

To provide a new leakage prevention bag (1) for injecting and filling the bone cement. With this leakage prevention bag, there is no danger of causing complications such as neuropathy, lung embolus or the like, which are caused by leakage of the bone cement injected into the vertebral body (4). Further, even when the bone cement (9) itself is not leaked, there is no organism damage done by the leakage of a monomer or a non-cured component from the bone cement (9) before curing, and the cement cannot contact blood or body fluid, thereby making an insufficient curing of the bone cement and decreasing the strength of the cured body. In this bone cement injecting and filling method, the leakage prevention bag (1) for injecting and filling the bone cement is made of a bioabsorbable material having a molecular weight of 5,000 to 500,000 and has a bag having the thickness of 10 to 800µm. An injection tube (2) is attached to an injection port for injecting the bone cement (9) of this leakage prevention bag (1), and this bag (1) is inserted into a cavity (5) formed in the vertebral body (4).

## Description

The present invention relates to a bone cement injecting and filling method and a leakage prevention bag for injecting and filling the bone cement in a percutaneous kyphoplasty. In this bone cement injecting and filling method, there is no danger of causing complication such as neuropathy, lung embolus or the like, where the complication is caused by leakage of the bone cement injected into a vertebral body to thereby compress nerves and internal organs around the bone cement. Further, there is no danger of causing other problems caused by leakage of the injected bone cement.

In accordance with advancing of an aging society, a patient having a vertebral compression fracture such as an osteoporotic spinal compression fracture or the like has been increasing, and patients having osteoporosis has been increasing also in the youth with the change of social structure or a lifestyle. The osteoporotic spinal compression fracture is a symptom of a disease where the vertebral body is made fragile to be crashed by weight or external force in accordance with advancing of the osteoporosis, and it is accompanied by a curve and pain of the spine.

As a conventional treating method, symptomatic treatments, such as a treatment by resting for a long time, a treatment by taking an analgesic and an antiosteoporotic agent, and a treatment by fixing an affected part with a hard corset (a gypsum), are mainly used. However, these treatments are not fundamental treatments. So, there are problems that a chronic pain remains, a motor function is decreased, a bedridden or depressed state is caused, or the like. Further, in a conservative treatment by fixing for a long period time using the hard corset, it is necessary to carry out an operation in many cases since in lumber portion of back pain remains or the neuropathy occurs due to an insufficient reset, an osteonecrosis, or a bone deformity.

Then, an operation, in which the bone cement having radiopasity is injected into the fractured vertebral body to thereby fix a compressed and fractured part (a percutaneous vertebroplasty), has been developed. This treatment comprises the steps of photographing the spine by MRI (a magnetic resonance imaging apparatus) to thereby confirm an affected part, carrying out a X-ray fluoroscopy to the affected part using angiograph or a computed tomograph, inserting an injection needle having a diameter of about 3 mm into the vertebral body while avoiding spinal nerves, and injecting the bone cement into the vertebral body to thereby reinforce and fix the fractured vertebral body (for example, refer to Japanese Patent Application Laid Open No. 2004-8510).

However, this treatment has a problem that the fractured vertebral body is fixed in the state of being crashed. So, an operation (a percutaneous kyphoplasty) comprising the steps of making a small hole in the compressed and crashed vertebral body from the back part, resetting it by using a specific jack, that is, resetting the compression-fractured bone to have an original shape, removing the jack, removing a fragile destroyed bone tissue in the vertebral body if necessary, and injecting a bone augmentation material such as the bone cement or the like into the vertebral body, has been used.

In this treatment, there is a method comprising the steps of making a cavity inside the compression-fractured vertebral body, expanding a balloon used as the jack in the cavity, and resetting the vertebral body to have the original shape. The balloon is made of a semi-elastic material such as polyethyene tere phthalate, silicone or the like, an elastic material such as latex, or a general flexible material (plastic, polyethylene, mylar, nylon, polyurethane, a metallic or composite material). This treatment using various kinds of balloon materials is for keeping a space for injecting the bone cement into the vertebral body by taking out the balloon after resetting the shape of the vertebral body (for example, refer to Japanese translation of PCT international application No. 2001-520530, 2003-529438).

As for both of the percutaneous vertebroplasty and the percutaneous kyphoplasty, there are few physical and psychological burdens of a patient, and these are excellent methods capable of removing the pain, rising in an early stage, and preventing the compressing and crashing of the vertebral body. However, when the vertebral body is compressed and crashed once, there are cracks or damages even when the resetting treatment is carried out. Thus, when the bone cement is injected, the injected bone cement leaks from the cracked or damaged part to the outside of the vertebral body. So, there is a danger of compressing the nerves and internal organs around the bone cement and to thereby cause the complication such as neuropathy, lung embolus or the like. In order to avoid this danger, an operator works carefully while confirming an injection condition by X-ray or the like, so that this work has a problem when the treatment is carried out.

Further, even when the treatment is finished without leakage of the bone cement from the cracks or the damages of the vertebral body, there are the following problems. As the bone cement, an acrylic bone cement, a calcium phosphate bone cement or the like is generally used, where the acrylic bone cement is composed mainly of a polyethylene methacrylate and a methacrylate monomer. So, in the case of using the acrylic bone cement, there is an organism damaging property by the leakage of the monomer or a non-cured component from the bone cement before curing. Further, in the case of using the calcium phosphate bone cement, when the cement is directly contacted with a tissue and blood or body fluid, it is cured in an excessive water content state. Thus, the curing of the calcium phosphate bone cement contacted with water becomes insufficient, and the strength of a cured body is decreased due to formation of a non-cured layer or occurrence of a crack.

Then, an objective of the present invention is to provide a new bone cement injecting and f il ling method and a leakage prevention bag for injecting and filling the bone cement, so that, in the percutaneous kyphoplasty, there is no danger of causing the complication such as neuropathy, lung embolus or the like by using this bone cement injecting and filling method, where the complication is caused by leakage of the bone cement injected into the vertebral body and compression thereby of nerves and internal organs around the bone cement at the time of injecting the bone cement. Further, even when the cement does not leak, there is no organism damaging property by the leakage of the monomer or a non-cured component from the bone cement before curing, in a case of using the acrylic bone cement. Further, in a case of using the calcium phosphate bone cement, there are no problems that the cement is directly contacted with a biotissue and blood or body fluid, to thereby make the curing of the calcium phosphate bone cement insufficient and decreasing the strength of the cured body due to the formation of a non-cured layer or the occurrence of a crack.

The earnest work was carried out in order to solve the above-mentioned problems and, as a result of this, the followings were found out. In order to prevent leakage of the bone cement from a cracked or damaged part of the vertebral body to the outside of it when the bone cement is injected, the bone cement is not directly injected into the vertebral body, but injected into a bag made of a bioabsorbable material in the vertebral body. Then, the bone cement does not leak from the cracked or damaged part of the vertebral body and does not affect to a living body irrespective of the kind of the bone cement. Further, since the bag is made of the bioabsorbable material, the bag is absorbed and decomposed after curing of the bone cement. Thus, even if the bag is not removed from the vertebral body, the problem does not occur.

That is, one aspect of the present invention is a bone cement injecting and filling method comprising the steps of forming a cavity inside a vertebral body to be treated; inserting an injection tube having an injection port of a leakage prevention bag for injecting and filling the bone cement, into a hole formed in the vertebral body so as to connect it with the cavity; and injecting and filling the bone cement into the leakage prevention bag for injecting and filling the bone cement in the cavity of the vertebral body through the tube. The leakage prevention bag comprises a bag made of the bioabsorbable material.

Further, the other aspect of the present invention is a leakage prevention bag for injecting and filling the bone cement, which is used in the bone cement injecting and filling method. This leakage prevention bag is made of the bioabsorbable material having a molecular weight of 5,000 to 500,000, and comprises a bag body having a thickness of 10 to 800 *µ* m and provided with an injection port for injecting and filling the bone cement.

Further, the followings were also found out. In the leakage prevention bag for injecting and filling the bone cement, it is preferable that the bag is made of a material which can be expanded. Further, it is also preferable that the bag is formed in a tube shape having an outmost diameter of 3 to 50 mm and a length of 10 to 200 mm, and one end of this body is closed and another end of this body has the injection port. Furthermore, it is preferable that the bioabsorbable polymer used as a raw material thereof is at least one kind selected from polylactic acid, polyglycolic acid, poly- ε- caprolactone, copolymer of lactic acid and glycolic acid, and copolymer of lactic acid and ε-caprolactone.

As for the bone cement injecting and filling method and the leakage prevention bag according to the present invention, when the bone cement is injected in the percutaneous kyphoplasty, it can be prevented to contact the bone cement with the vertebral body at least until the injected bone cement has cured. Thus, there is no danger of causing the complication such as neuropathy, lung embolus or the like, where the complication is caused by leakage of the bone cement injected into the vertebral body and compression thereby of nerves and internal organs around the bone cement. Further, there are no conventional problems that the bone cement leaks from the cracked or damaged part of the vertebral body, and the curing of the bone cement is insufficient. Thus, the bone cement injecting and filling method and the leakage prevention bag for injecting and filling the bone cement according to the present invention are excellent, and have a great value for contributing to the medical treatment.
Figure 1 is a perspective view showing one example in a state where an injection port of a leakage prevention bag for injecting and filling a bone cement according to the present invention is mounted to an injection tube.
Figures 2 to 5 show medical treatment processes carried out before injecting and filling the bone cement according to the present invention in the percutaneous kyphoplasty.

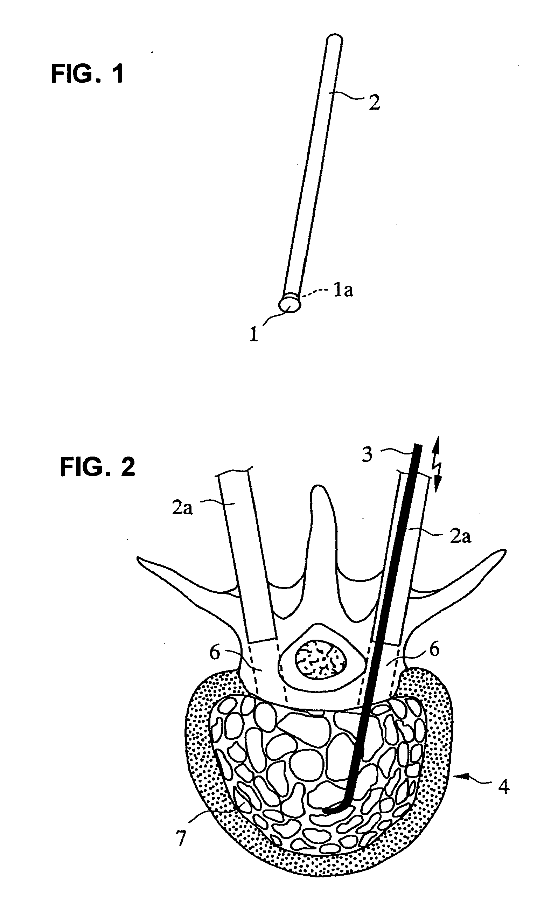

Figure 2 is an explanation view showing a state where two holes are bored in the vertebral body, and a specific crushing tool is inserted from one hole into the vertebral body.

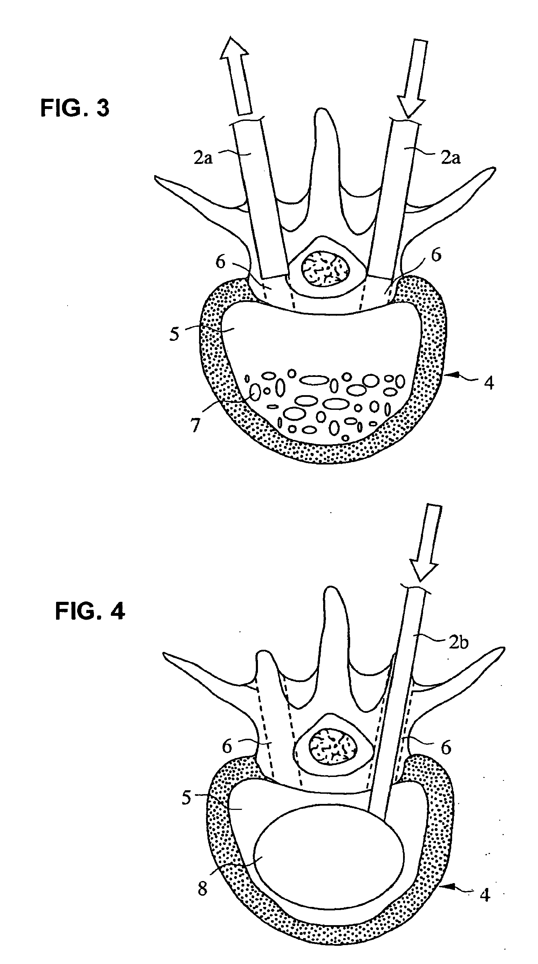

Figure 3 is an explanation view showing a state where a crushed bone structure in the vertebral body is sucked, removed and to thereby form a cavity.
Figure 4 is an explanation view showing a state where a balloon capable of expanding is inserted into the cavity of the vertebral body as a jack for shaping and expanded in the cavity.

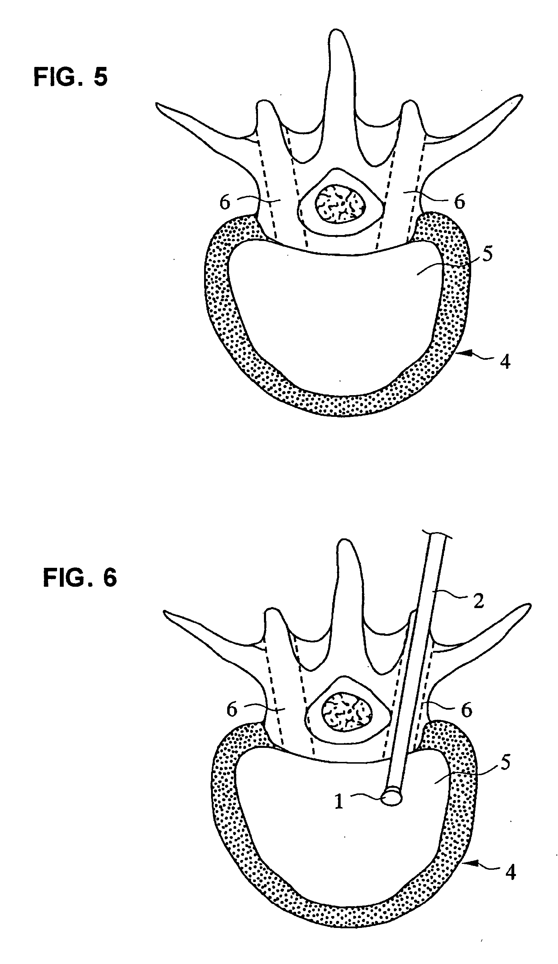

Figure 5 is an explanation view showing a state where the balloon is shrunk and removed after completely expanding and finishing the correction of the cavity.
Figures 6 to 9 show one example of medical treatment processes in which the leakage prevention bag for injecting and filling of the bone cement, which is shown in Figure 1, is used to carry out injecting and filling the bone cement according to the present invention in the percutaneous kyphoplasty.
Figure 6 is an explanation view showing a state where the injection tube shown in Figure 1 is inserted in the cavity of the vertebral body, the tube being attached with the injection port of the leakage prevention bag for injecting and filling a bone cement according to the present invention.

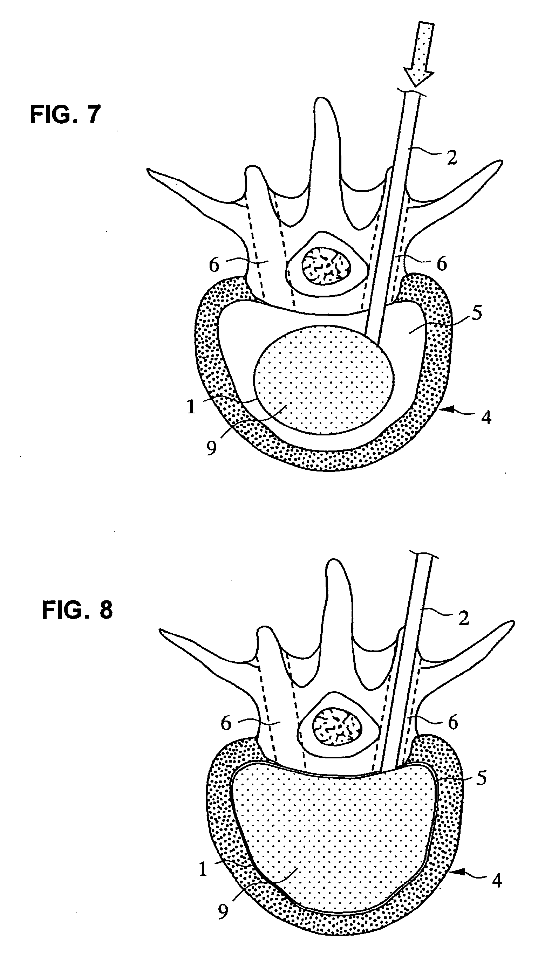

Figure 7 is an explanation view showing a state where the bone cement is injected and filled into the leakage prevention bag for injecting and filling a bone cement through the injection tube.
Figure 8 is an explanation view showing a state where injecting and filling of the bone cement is finished.

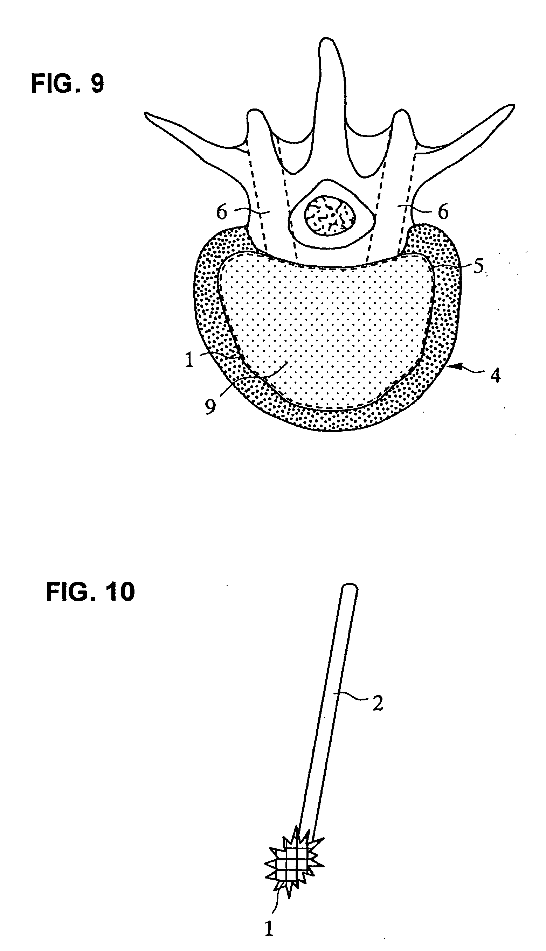

Figure 9 is an explanation view showing a state where the injection tube is removed from the vertebral body.
Figure 10 is a perspective view showing another example in a state where an injection port of a leakage prevention bag for injecting and filling a bone cement according to the present invention is mounted to an injection tube.

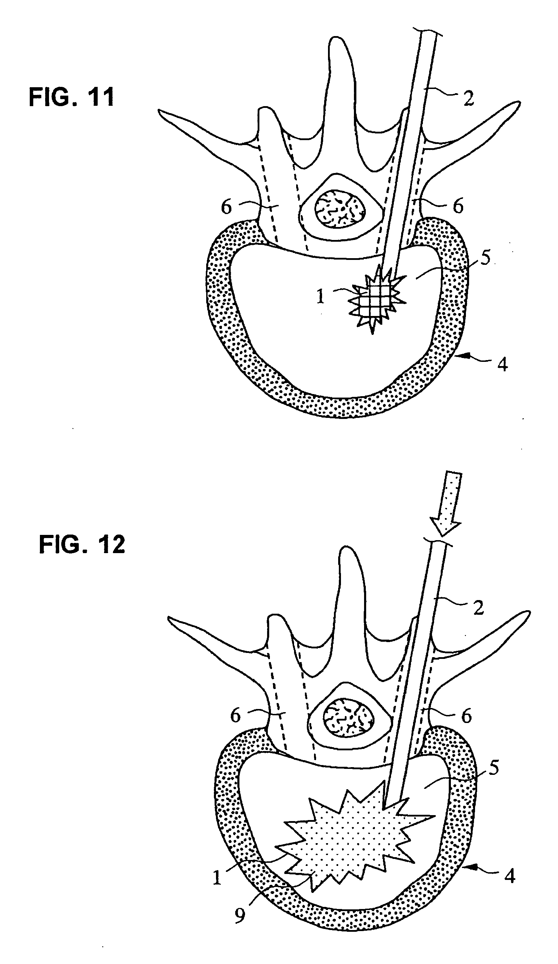

Figures 11 to 12 show one example of medical treatment processes in which the leakage prevention bag for injecting and filling of the bone cement, which is shown in Figure 10, is used to carry out injecting and filling the bone cement according to the present invention in the percutaneous kyphoplasty.
Figure 11 is an explanation view showing a state where the injection tube shown in Figure 10 is inserted in the cavity of the vertebral body, the tube being attached with the injection port of the leakage prevention bag for injecting and filling a bone cement according to the present invention.
Figure 12 is an explanation view showing a state where the bone cement is injected and filled into the leakage prevention bag for injecting and filling a bone cement through the injection tube.

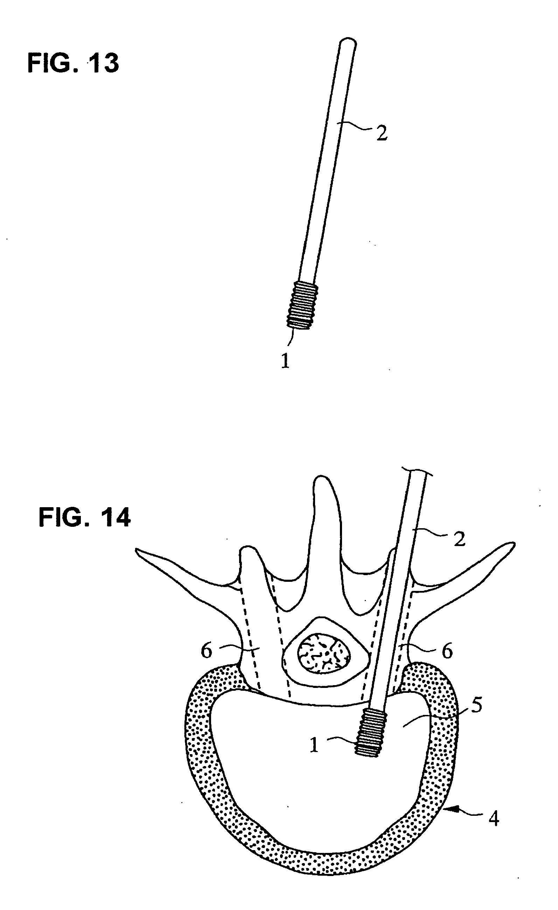

Figure 13 is a perspective view showing another example in a state where an injection port of a leakage prevention bag for injecting and filling a bone cement according to the present invention is mounted to an injection tube.
Figures 14 to 16 show one example of medical treatment processes in which the leakage prevention bag for injecting and filling of the bone cement, which is shown in Figure 13, is used to carry out injecting and filling the bone cement according to the present invention in the percutaneous kyphoplasty.
Figure 14 is an explanation view showing a state where the injection tube shown in Figure 13 is inserted in the cavity of the vertebral body, the tube being attached with the injection port of the leakage prevention bag for injecting and filling a bone cement according to the present invention.

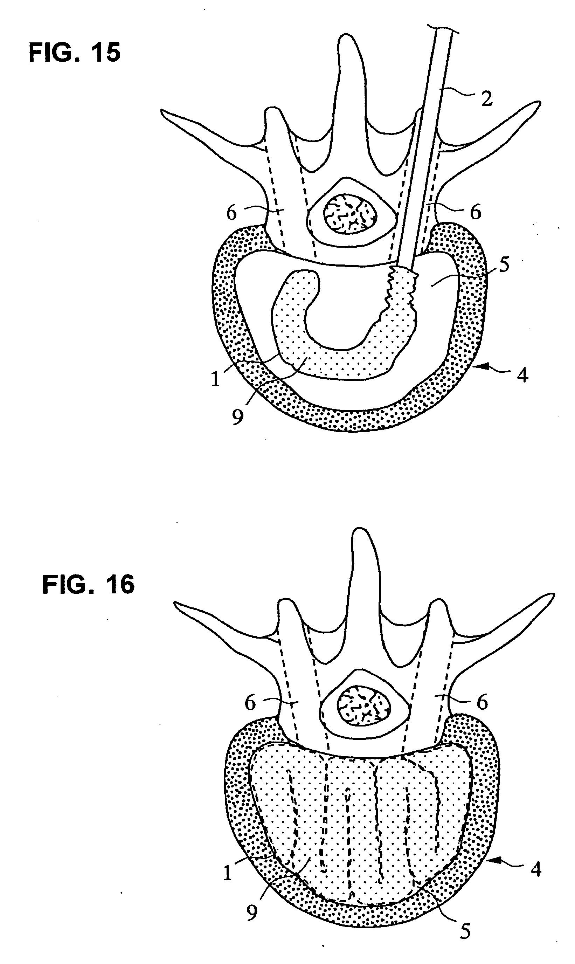

Figure 15 is an explanation view showing a state where the bone cement is injected and filled into the leakage prevention bag for injecting and filling a bone cement through the injection tube.
Figure 16 is an explanation view showing a state where injecting and filling of the bone cement is almost finished and the injection tube is removed from the vertebral body.

Hereinafter, the bone cement injecting and filling method according to the present invention, and the leakage prevention bag for injecting and filling the bone cement used for this method are explained concretely with drawings.

In the drawings, a reference numeral 1 is a leakage prevention bag for injecting and filling the bone cement according to the present invention, and this bag 1 is made of the bioabsorbable material having the molecular weight of 5,000 to 500,000. As for a kind of the bioabsorbable material, it is not especially limited if it can be processed to have a film state and has high biocompatibility. However, if the bioabsorbable material is at least one kind selected from polylactic acid, polyglycolic acid, poly- ε- caprolactone, copolymer of lactic acid and glycolic acid, and copolymer of lactic acid and ε- caprolactone, it is preferable on stability and safety of the material. In these materials, polylactic acid, polyglycolic acid and copolymer of lactic acid and glycolic acid are approved as a polymer being harmless to the human body by the U. S . Food and Drug Administration, so that these materials are especially preferable. Thus, the most preferable material may be used according to the material of the bone cement. For example, the polylactic acid may be selected in the case of using the acrylic bone cement, and copolymer of lactic acid and glycolic acid may be selected in the case of using the calcium phosphate bone cement. In addition, a material derived from an animal is not preferable from viewpoint of contamination of xenogenic protein.

As described above, the bioabsorbable material to make the leakage prevention bag for injecting and filling the bone cement 1 has a weight average molecular weight of 5,000 to 500,000. When the molecular weight is less than 5,000, there are problems in moldability and strength. When the molecular weight is more than 500,000, it is hard to synthesize the high polymer and is necessary too much time- for dissolving and absorbing. So, it is not preferable.

The leakage prevention bag for injecting and filling the bone cement 1 comprises a bag body having at least one injectionport 1a for injecting and filling the bone cement into the inside thereof. The thickness of the bag body is 10 to 800 *µ*m. When the thickness is less than 10 *µ*m, the bag 1 cannot have a sufficient strength to resist the pressure at the time of injecting and filling. When the thickness is more than 800 *µ* m, the bag is too hard, so that adhesiveness to the vertebral body in the cavity and operativity at the time of passing through a thin tube are lowered, and, further, it takes too much time for dissolving and absorbing the leakage prevention bag. In addition, when the bag 1 is used while being expanded by injecting and filling of the bone cement like a balloon, the injecting pressure is increased too much. So, it is not preferable. It is more preferable that the thickness is 50 to 500 *µ*m.

As for the shape of the leakage prevention bag for injecting and filling the bone cement 1, it may have various shapes according to an applied portion of the vertebral body and is not limited especially. For example, it may be of various shapes, such as a spherical shape, various kinds of disc shapes, various kinds of cylindrical shapes, a football shape, a doughnut shape, a conical shape, a waterdrop shape, a star shape or the like, and a complex shape of these shapes. Further, the shape may be specially made according to a shape of each affected part, which is different from predetermined shapes. Furthermore, when the bag body has a complicated complex shape, the inside of the bag may be subdivided and provided with plural injection ports 1a.

When the leakage prevention bag for injecting and filling the bone cement 1 is made of the material capable of expanding as illustrated in Figure 1, the bag 1 can easily correspond to the shape of a cavity wall in the vertebral body, so it is more preferable.

Further, as for the leakage prevention bag for injecting and filling the bone cement 1, it is preferable that the bag body may be pre-formed in a specific shape and folded in a manner of being able to be unfolded according to its necessity when injecting and filling the bone cement, as illustrated in Figure 10. Further, it is also preferable that the bag may have the tube shape having the outmost diameter of 3 to 50 mm and the length of 10 to 200 mm, as illustrated in Figure 13. In such cases, the bone cement can be injected and filled comparatively freely in the cavity formed in the vertebral body, and the bag 1 can be suited to various shapes of the cavity. When the outmost diameter is less than 3 mm or the length is less than 10 mm, it is hard to form the bag and to inject and fill the bone cement. Further, when the outmost diameter is more than 5 mm or the length is more than 200 mm, the operativity may be lowered.

Further, when the leakage prevention bag for injecting and filling the bone cement 1 has the tube shape, the injection port 1a may be provided on the side surface of the tube and the bone cement may be injected and filled from the injection port 1a into plural tubes having closed ends. Further, one end of the tube may be closed, and another end may have the injection port 1a.

In order to carry out the percutaneous kyphoplasty by using such the leakage prevention bag for injecting and filling the bone cement 1, at first, the medical treatment for resetting the fractured or crushed vertebral body is carried out to have the original shape. This medical treatment comprises the following 4 processes. A first process comprises the steps of boring two holes 6, 6 in a vertebral body 4 which must be treated; inserting guide tubes 2a, 2a through the holes 6, 6; and pulverizing a bone trabecula 7 inside the vertebral body 4 by using a specific crushing tool 3 through the guide tube 2a which is inserted into the hole 6, if necessary, as illustrated in Figure 2. A second process comprises the steps of injecting a physiological saline from the guide tube 2a; sucking and removing the pulverized bone trabecula 7 inside the vertebral body, and forming a cavity 5, as illustrated in Figure 3. A third process comprises the steps of inserting a non-bioabsorbable balloon 8 into the formed cavity 5 through the hole 6 using the specific tube 2b; and expanding the balloon 8 by air, as illustrated in Figure 4, where the balloon 8 can be expanded as the jack for shaping. A fourth process comprises the steps of resetting the fractured or crushed vertebral body to have the original shape by expanding the balloon 8 in the cavity 5; shrinking the balloon 8 completely; and removing the balloon 8 from the inside of the cavity 5 with the specific tube 2b, as illustrated in Figure 5. In addition, depending on the strength of the vertebral body 4, it is possible to apply the steps comprising directly inserting the non-bioabsorbable balloon 8 into the vertebral body 4 without using the crushing tool 3; expanding the balloon; compressing the bone trabecula 7 and a spongy bone toward the inside surface of a cortical bone; and thus expanding the cavity 5. Further, although it is not illustrated in the drawings, for example, when the bone trabecula 7 is comparatively weak, a smaller type non- bioabsorbable balloon may be used to compress the bone trabecula 7, to thereby form the small cavity 5 or a passage at first, and then, the non-bioabsorbable balloon 8 may be inserted to compress the bone trabecula 7 toward ₜₕₑ surrounding. Such processes are the same as processes in the conventional percutaneous kyphoplasty.

At this time, the non-bioabsorbable balloon 8 is made of a non-elastic material (a flexible material such as polyethylene terephthalate (PET)) in order not to give the external force more than needed to the vertebral body 4, and the balloon 8 is used by calculating to have the needed size after expanding.

After finishing the medical treatment for resetting the fractured or crushed vertebral body 4 to have the original shape in this way, the bone cement injecting and filling method according to the present invention is carried out as follows.

As illustrated in Figures 6, 11 and 14, the injection tube 2 having the leakage prevention bag for injecting and filling the bone cement 1 at the tip end thereof is inserted into the cavity 5 formed in the vertebral body 4 through the hole 6. After that, as illustrated in Figures 7, 12 and 15, the bone cement 9 is injected and filled into the leakage prevention bag for injecting and filling the bone cement 1 from the injection tube 2. In the case of the embodiment illustrated in Figure 7, the folded leakage prevention bag is expanded. In the case of the embodiment illustrated in Figure 12, the folded leakage prevention bag 1 is unfolded. In the case of the embodiment illustrated in Figure 15, the leakage prevention bag 1 is : pulled out successively. Thereby, as illustrated in Figures 8 and 16, the leakage prevention bag 1 is appropriately suited corresponding to the shape of the cavity 5. After that, as illustrated in Figures 9 and 16, the leakage prevention bag 1 is detached from the injection tube 2, and the injection tube 2 is removed from the inside of the living body. Then, an incision part of the skin is covered with a bandage, to thereby complete the medical operation.

### [Example]

### Example 1

As the leakage prevention bag for injecting and filling the bone cement 1 according to the present invention to be used in the bone cement injecting and filling method according to the present invention and as illustrated in Figures 6 to 9, a leakage prevention bag made of copolymer of lactic acid and glycolic acid (weight-average molecular weight was 250,000 and weight ratio was 75: 25), which was a bioabsorbable polymer material, and provided with one bone cement injection port 1a having a diameter of 3 mm and a bag body capable of expanding to about 240% at a surface area and having an average thickness before expanding of about 500 *µ*m and a diameter of about 4 mm was produced.

A calcium phosphate bone cement (the trade name was Biopex produced by Mitsubishi Material Corporation) was injected and filled into the leakage prevention bag 1 until expanding to about 200% at the surface area. Leakage of the calcium phosphate bone cement to the outer surface of the leakage prevention bag 1 was not observed until the calcium phosphate bone cement was cured.

### Example 2

As the leakage prevention bag for injecting and filling the bone cement 1 according to the present invention to be used in the bone cement injecting and filling method according to the present invention and as illustrated in Figures 11 to 12, a leakage prevention bag made of the polylactic acid (weight-average molecular weight was 280,000), which was the bioabsorbable polymer material, and provided with one bone cement injection port 1a having the diameter of 3 mm and a bag, which had a cylindrical shape before folding and which had about 32 mm diameter, about 10 mm length and about 2 0 0 /₁ m thickness, was capable of expanding to about 180% at the surface area, and had a cylindrical shape after folding with about 3 mm diameter and about 8 mm length, was produced.

An acrylic bone cement (the trade name was Surgicalsynblex produced by Nippon Striker Corporation) was injected and filled into the leakage prevention bag for injecting and filling the bone cement 1 until expanding to about 150% at the surface area. Leakage of the acrylic bone cement to the outer surface of the leakage prevention bag was not observed until the acrylic bone cement was hardened.

### Example 3

As the leakage prevention bag for injecting and filling the bone cement 1 according to the present invention to be used in the bone cement injecting and filling method according to the present invention and as illustrated in Figures 14 to 16, a leakage prevention bag made of copolymer of lactic acid and poly- ε-caprolactone (weight-average molecular weight was 390,000 and the weight ratio was 75: 25), which was the bioabsorbable polymer material, and provided with one bone cement injection port 1a having the diameter of 3 mm and a tube-like bag body, which had the diameter of about 8 mm and the length of about 200 mm, was closed at another end, had the thickness of about 160 *µ*m, was capable of expanding to about 200% at the surface area and was folded in a bellows shape at a position being 10 mm from the tip end of the injection tube 2, was produced.

A calcium phosphate bone cement (the trade name was Biopex produced by Mitsubishi Material Corporation) was injected and filled into the leakage prevention bag 1 until expanding to about 180% at the surface area. Leakage of the calcium phosphate bone cement to the outer surface of the leakage prevention bag 1 was not observed until the calcium phosphate bone cement was hardened.

As for the leakage prevention bag for injecting and filling the bone cement of each example, the bone cement does not leak at least until the bone cement is injected, filled, and hardened. Thus, in the percutaneous kyphoplasty, there is no danger of causing the complication disease such as neuropathy, lung embolus or the like, where the complication is caused by leakage of the bone cement injected into the vertebral body and compression of nerves and internal organs around the bone cement. Further, there are no problems that the bone cement leaks from the cracked or damaged part of the vertebral body and the hardening of the bone cement is insufficient.

## Claims

1. Abone cement injecting and filling method, the method comprising the steps of;
forming a cavity 5 inside a vertebral body 4 which must be treated;
inserting an injection tube 2 having an injection port 1a of a leakage prevention bag for injecting and filling bone cement 1, into a hole 6 formed in the vertebral body 4 in order to form the cavity 5, wherein the leakage prevention bag 1 comprises a bag body made of a bioabsorbable material; and injecting and filling a bone cement 9 into the leakage prevention bag 1 in the cavity 5 through the injection tube 2.

2. A leakage prevention bag for injecting and filling a bone cement 1,
wherein said leakage prevention bag 1 is made of the bioabsorbable material having a molecular weight of 5, 000 to 500, 000, and comprises a bag having a thickness of 10 to 800 *µ*m and provided with an injection port 1a for injecting and filling the bone cement 9.

3. The leakage prevention bag for injecting and filling a bone cement 1 according to claim 2,
wherein the bag body is made of a material capable of expanding.

4. The leakage prevention bag for injecting and filling a bone cement 1 according to claim 2 or 3,
wherein the bag body before injecting and filling the bone cement 9 is formed in a tube shape having an outmost diameter of 3 to 50 mm and a length of 10 to 200 mm.

5. The leakage prevention bag for injecting and filling a bone cement 1 according to claim 4,
wherein one end is closed and another end has the injection port 1a.

6. The leakage prevention bag for injecting and filling a bone cement 1 according to any one of claims 2 to 5,
wherein the bioabsorbable material is at least one kind selected from polylactic acid, polyglycolic acid, poly- ε- caprolactone, copolymer of lactic acid and glycolic acid, and copolymer of lactic acid and ε-caprolactone.

7. Use of a leakage prevention bag for injecting and filling a bone cement according to one or more of claims 2 to 6 to form a bone cement filling in a cavity of a vertebral body.
